# EUROPEAN PATENT APPLICATION

(11) **EP 3 987 925 A1**
(43) Date of publication of application: **27.04.2022**
(21) Application number: 20825592.7
(22) Date of filing: 04.06.2020
(51) Int. Cl.: A01K 29/00, A01K 11/00, A61B 5/107

(54) **ANIMAL INFORMATION MANAGEMENT SYSTEM AND ANIMAL INFORMATION MANAGEMENT METHOD**

(30) Priority: 21.06.2019 JP 2019115368
(71) Applicant: Panasonic Intellectual Property Management Co., Ltd., Osaka-shi, Osaka 540-6207 (JP)
(72) Inventor: FUJIYAMA, Hiromitsu, Osaka-shi, Osaka 540-6207 (JP); INABA, Yuichi, Osaka-shi, Osaka 540-6207 (JP)
(74) Representative: Appelt, Christian W.
(86) International application number: PCT/JP2020/022205
(87) International publication number: WO 2020/255742

(57) **Abstract**

An animal information management system (100) includes a storage (24) and an information processor (22). The information processor (22) causes an imaging device (10) to perform tracking imaging of a pig (80), and accumulates still pictures including the pig (80) in the storage (24), the still pictures being obtained during the tracking imaging. The information processor (22) brings the imaging device (10) into a zoom-in state having a zooming magnification higher than that during the tracking imaging when a predetermined requirement for the accumulated still pictures is satisfied, obtains the identification information of the pig (80) by causing the imaging device (10) in the zoom-in state to capture an image of the pig (80), and stores the identification information in the storage (24), in association with the accumulated still pictures.

## Description

### [Technical Field]

The present disclosure relates to an animal information management system and an animal information management method.

### [Background Art]

Techniques of identifying animal individuals and managing the states of growth of the animals or their health conditions are known in the related art. PTL 1 discloses a biological information processing apparatus which identifies animal individuals using images.

### [Citation List]

### [Patent Literature]

[PTL 1] Japanese Unexamined Patent Application Publication No. 2018-007625

### [Summary of Invention]

### [Technical Problem]

The present disclosure provides an animal information management system and an animal information management method which enable efficient identification of animal individuals using still pictures.

### [Solution to Problem]

The animal information management system according to one aspect of the present disclosure includes: a storage; and an information processor, wherein the information processor: causes an imaging device to perform tracking imaging of an animal; accumulates still pictures including the animal in the storage, the still pictures being obtained during the tracking imaging; brings the imaging device into a zoom-in state having a zooming magnification higher than a zooming magnification during the tracking imaging when a predetermined requirement for the still pictures accumulated is satisfied, and obtains identification information of the animal by causing the imaging device in the zoom-in state to capture an image of the animal; and stores the identification information in the storage, in association with the still pictures.

The animal information management method according to one aspect of the present disclosure is an animal information management method to be executed by a computer, the animal information management method including: causing an imaging device to perform tracking imaging of an animal; accumulating still pictures including the animal in a storage, the still pictures being obtained during the tracking imaging; bringing the imaging device into a zoom-in state having a zooming magnification higher than a zooming magnification during the tracking imaging when a predetermined requirement for the still pictures is satisfied, and obtaining identification information of the animal by causing the imaging device in the zoom-in state to capture an image of the animal; and storing the identification information in the storage, in association with the still pictures accumulated.

### [Advantageous Effects of Invention]

The animal information management system and the animal information management method according to the present disclosure enable efficient identification of animal individuals using still pictures.

### [Brief Description of Drawings]

[FIG. 1]

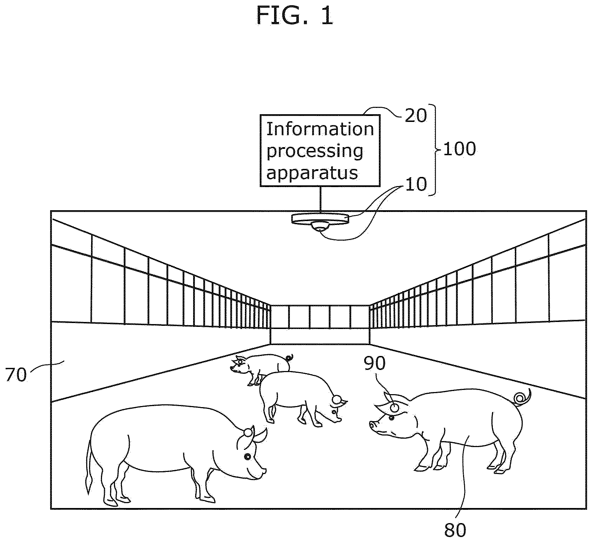

   FIG. 1 is a diagram illustrating a schematic configuration of the animal information management system according to an embodiment.
[FIG. 2]

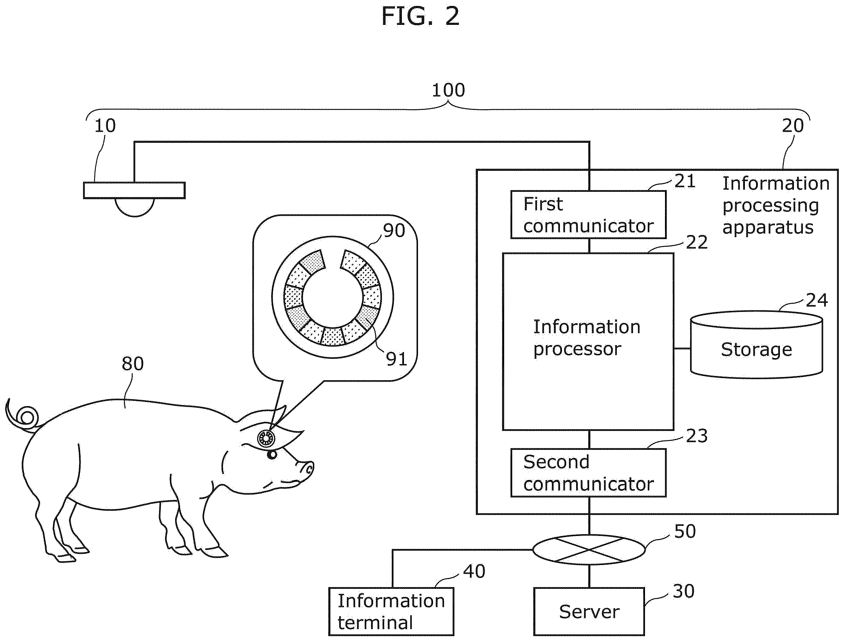

   FIG. 2 is a block diagram illustrating a functional configuration of the animal information management system according to the embodiment.
[FIG. 3]

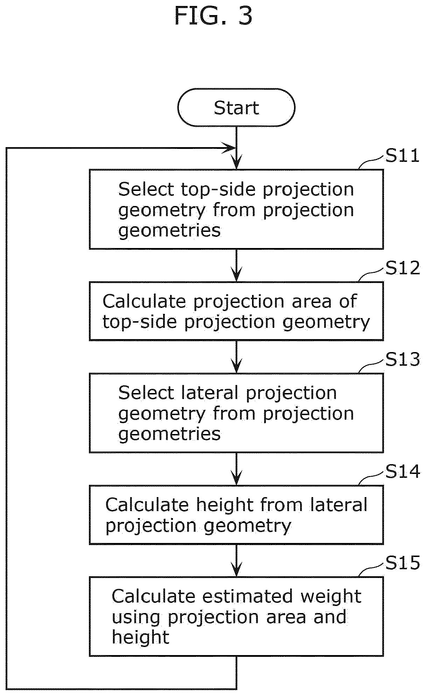

   FIG. 3 is a flowchart of a method of estimating the weight of a pig.
[FIG. 4]

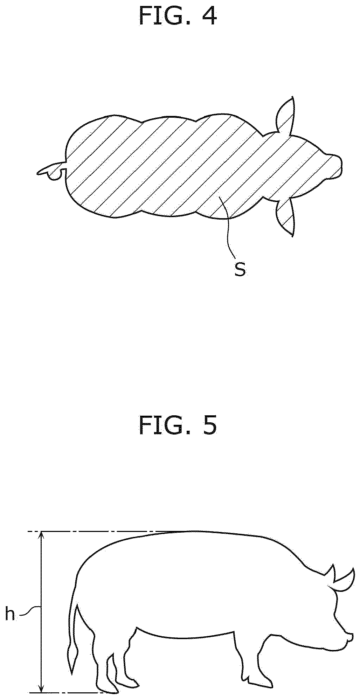

   FIG. 4 is a diagram illustrating one example of a top-side projection geometry.
[FIG. 5]
   FIG. 5 is a diagram illustrating one example of a lateral projection geometry.
[FIG. 6]

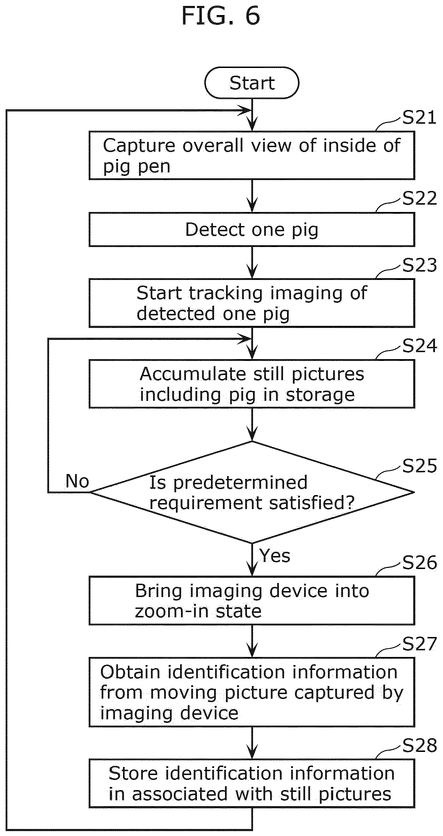

   FIG. 6 is a flowchart of Operational Example 1 in the animal information management system according to the embodiment.
[FIG. 7]

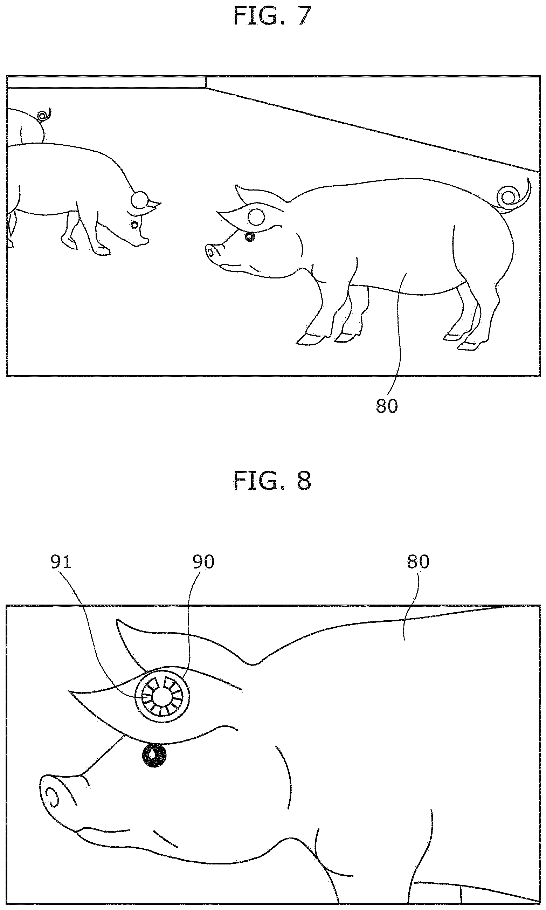

   FIG. 7 is a diagram illustrating one example of a moving picture obtained during tracking imaging.
[FIG. 8]
   FIG. 8 is a diagram illustrating one example of the moving picture captured by the imaging device in the zoom-in state.
[FIG. 9]

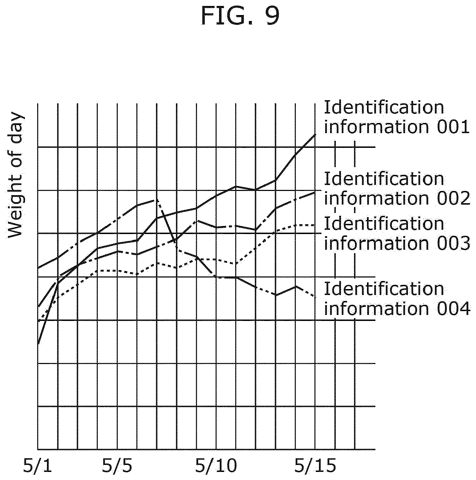

   FIG. 9 is a diagram illustrating one example of the graph of the weight.
[FIG. 10]

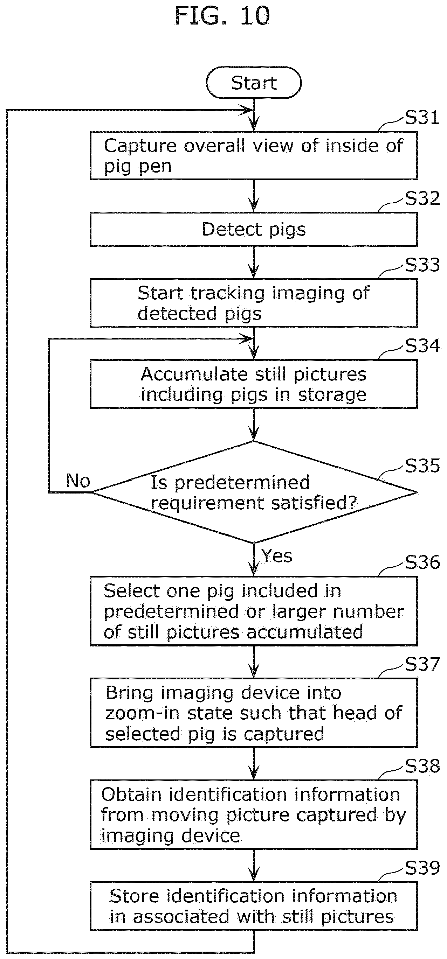

   FIG. 10 is a flowchart of Operational Example 2 in the animal information management system according to the embodiment.
[FIG. 11]

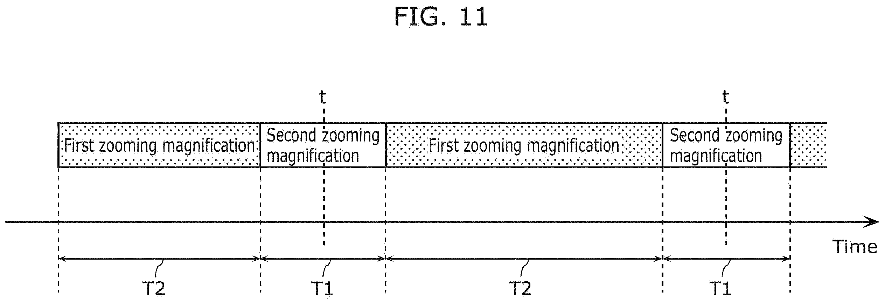

   FIG. 11 is a diagram illustrating one example of a change in zooming magnification during tracking imaging.
[FIG. 12]

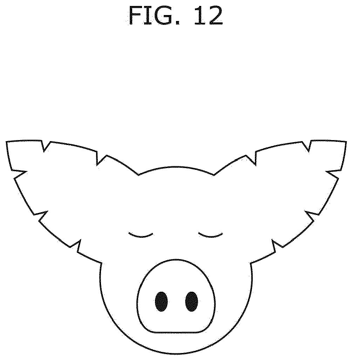

   FIG. 12 is a diagram illustrating one example of ear notching.

### [Description of Embodiments]

An embodiment will now be described with reference to the drawings. The embodiments described below all illustrate comprehensive or specific examples. Numeric values, shapes, materials, components, arrangements and positions of the components, and connections forms thereof shown in the embodiment below are illustrative, and should not be construed as limitations to the present disclosure. Moreover, among the components of the embodiments below, the components not described in an independent claim will be described as optional components.

The drawings are schematic views, and are not necessarily precise illustrations. In the drawings, identical reference sings are given to substantially identical configurations, and overlapping description will be omitted or simplified in some cases.

### [Embodiment]

### [Configuration]

The animal information management system according to an embodiment will now be described with reference to the drawings. First, the configuration of the animal information management system according to the embodiment will be described. FIG. 1 is a diagram illustrating the schematic configuration of the animal information management system according to the embodiment. FIG. 2 is a block diagram illustrating the functional configuration of the animal information management system according to the embodiment.

As illustrated in FIG. 1, animal information management system 100 is a system which can individually manage pieces of information on a plurality of pigs 80 in pig pen 70 based on an image of an inside of pig pen 70 captured by imaging device 10. Pig pen 70 is one example of a livestock barn, and pig 80 is one example of an animal (farm animal). The raiser of pig 80 can know the growth states of pigs 80 using animal information management system 100. As illustrated in FIG. 2, animal information management system 100 includes imaging device 10 and information processing apparatus 20. FIG. 2 also illustrates server 30 and information terminal 40.

Imaging device 10 is a camera attached on the ceiling of pig pen 70 or the like to capture the overall view of the inside of pig pen 70. Imaging device 10 has pan, tilt, and zoom functions. Imaging device 10 is implemented with a lens and an image sensor, for example. Imaging device 10 may be an omni-directional camera. Imaging device 10 may have a measurement function according to a time of flight (TOF) method (more specifically, a function to measure the distance from imaging device 10 to the target to be captured).

Based on the image captured by imaging device 10, information processing apparatus 20 can identify a plurality of pigs 80 present inside pig pen 70, and can output individual growth information for each of pigs 80. The individual growth information is an estimated weight, for example, and may be an estimated amount of movement or the like. Information processing apparatus 20 includes first communicator 21, information processor 22, second communicator 23, and storage 24.

First communicator 21 is a communication circuit (communication module) for information processing apparatus 20 to perform communication with imaging device 10. First communicator 21 obtains an image captured by imaging device 10 (or picture data), for example. The communication performed by first communicator 21 may be wired communication or may be wireless communication. First communicator 21 can communicate according to any communication standards.

Information processor 22 controls imaging device 10 through first communicator 21. Information processor 22 also performs information processing using the image captured by imaging device 10 to estimate the individual growth information for each of pigs 80 present in pig pen 70. Information processor 22 is implemented with a microcomputer, for example, and may be implemented with a processor.

Second communicator 23 is a communication circuit (communication module) for information processing apparatus 20 to communicate with other apparatuses through a wide area communication network such as the Internet 50. For example, second communicator 23 transmits the individual growth information of each of pigs 80 to server 30 or information terminal 40. Transmission of such individual growth information to information terminal 40 of the raiser enables the raiser to know the growth states of pigs 80 inside pig pen 70. The individual growth information may be transmitted to information terminal 40 via server 30. Server 30 may be connected to information terminal 40 through a communication network such as a local area network (LAN). In this case, the raiser can also use information terminal 40 within the LAN. The communication performed by second communicator 23 may be wired communication or may be wireless communication. Second communicator 23 can communicate according to any communication standards.

Storage 24 is a memory device which stores programs executed by information processor 22 to perform the information processing and a variety of pieces of information used in the information processing. Storage 24 is specifically implemented by a semiconductor memory.

Identification tags 90 are attached to pigs 80 inside pig pen 70. Identification tag 90 is a disk-shaped member used to identify each individual pig 80, and is made of a polyurethane resin, for example. Identification tag 90 includes color code 91 which indicates the identification information (or ID) of pig 80 by a sequence of mutually different adjacent colors.

Color code 91 indicates the identification information of pig 80 by a sequence of mutually different adjacent colors (e.g., three colors of red (R), green (G), and blue (B)) excluding white and black. In color code 91, the bit value is determined according to the adjacent colors. For example, when the adjacent colors in color code 91 change from red to blue, from blue to green, and from green to red, the bit value is "1". When the adjacent colors in color code 91 change from red to green, from green to blue, and from blue to red, the bit value is "0". Thus, the rule for identifying color code 91 as digital data is preliminarily specified. Although the mutually different adjacent colors are aligned in the form of a character C (or a Landolt ring) in identification tag 90, the mutually different adjacent colors may be linearly aligned, or may be aligned in a matrix.

### [Estimation of weight]

Information processor 22 can estimate the weight of pig 80, for example, based on a projection geometry of pig 80 in a still picture captured by imaging device 10. First, one example of a method of estimating the weight of pig 80 will be described. FIG. 3 is a flowchart of the method of estimating the weight of pig 80.

Information processor 22 can obtain the projection geometry of pig 80 by specifying a portion of the still picture which includes pig 80, and performing contour extraction on the specified portion. For example, information processor 22 obtains a plurality of projection geometries from a plurality of still pictures captured by imaging device 10, and selects a projection geometry viewed from above (hereinafter, simply referred to as, top-side projection geometry) from the plurality of projection geometries (S11). Specifically, information processor 22 selects a top-side projection geometry using a machine learning model or the like. FIG. 4 is a diagram illustrating one example of the top-side projection geometry.

Next, information processor 22 calculates projection area S of the top-side projection geometry (S12). Each still picture is associated with the capturing condition information of imaging device 10 when the still picture is captured, such as the pan angle, the tilt angle, and the zooming magnification. Information processor 22 can calculate projection area S by converting the lengths in the still picture to their actual lengths based on the capturing condition information and position information of pig 80 included in the still picture.

Next, information processor 22 selects a projection geometry viewed from a lateral side (hereinafter, simply referred to as lateral projection geometry) from the plurality of projection geometries (S13). Specifically, information processor 22 can select a lateral projection geometry using a machine learning model or the like. FIG. 5 is a diagram illustrating one example of the lateral projection geometry. Information processor 22 calculates height h from the lateral projection geometry (S14). As described above, each still picture is associated with the capturing condition information of imaging device 10 when the still picture is captured, such as the pan angle, the tilt angle, and the zooming magnification. Information processor 22 can calculate height h by converting the lengths in the still picture to their actual lengths based on the capturing condition information and the position information of pig 80 included in the still picture.

Storage 24 stores a function or data table for calculating the estimated weight using projection area S and height h. Information processor 22 can calculate (estimate) the weight of pig 80 using such a function or data table (S15).

Any weight estimation method other than the above method can be used. Information processor 22 may estimate the weight by an image processing technique from three or more still pictures of pig 80 captured from different observing points, for example.

### [Operational Example 1]

To estimate the weight as described above, for each of pigs 80 inside pig pen 70, imaging device 10 needs to capture a still picture from which the top-side projection geometry can be obtained and a still picture from which the lateral projection geometry can be obtained. Thus, Operational Example 1 of animal information management system 100 for capturing such still pictures will be described. FIG. 6 is a flowchart of Operational Example 1 of animal information management system 100.

Initially, imaging device 10 captures the overall view of the inside of pig pen 70 (S21). Information processor 22 detects any one pig 80 by contour extraction performed on a moving picture during image capturing (S22), and starts tracking imaging of the pig detected by imaging device 10 (S23).

The term "tracking imaging" indicates an image capturing method in which at least one of the pan angle, the tilt angle, and the zooming magnification of imaging device 10 is controlled such that the entire body of pig 80 for tracking is always captured within the capturing range of imaging device 10 even when pig 80 for tracking moves inside pig pen 70. FIG. 7 is a diagram illustrating one example of the moving picture obtained during tracking imaging. Such tracking imaging can be implemented by an existing algorithm such as an approach using artificial intelligence. The zooming magnification during tracking imaging is set such that pig 80 is captured as large as possible within the capturing range (i.e., within the still picture). The zooming magnification during tracking imaging is fixed, for example.

Next, information processor 22 accumulates still pictures, which include pig 80, in storage 24 during tracking imaging (S24). For example, information processor 22 performs contour extraction on pig 80 for tracking in the moving picture obtained during tracking imaging, and when it is determined that a contour extracted using a machine learning model or the like corresponds to the top-side projection geometry of pig 80, information processor 22 stores the still pictures including such a contour in storage 24. Similarly, when it is determined that a contour extracted using a machine learning model or the like corresponds to the lateral projection geometry thereof, information processor 22 stores the still pictures including such a contour in storage 24.

Thus, in step S24, the still pictures enabling extraction of the top-side projection geometry and those enabling extraction of the lateral projection geometry are accumulated. Some weight estimation methods may need a projection geometry other than the top-side projection geometry and the lateral projection geometry in some cases. In such a case, another projection geometry may be accumulated in step S24.

In step S24, information processor 22 associates each of the still pictures with the capturing condition information of imaging device 10 when the still picture is captured, such as the pan angle, the tilt angle, and the zooming magnification. Such capturing condition information is one example of the position information indirectly indicating the position of pig 80 for tracking inside pig pen 70. In step S24, information processor 22 may specify coordinates information on pig 80 for tracking inside pig pen 70 based on the capturing condition information and the position information of pig 80 for tracking within each of the still pictures, and may associate the still pictures with the specified coordinates information. Such coordinates information is one example of the position information indirectly indicating the position of pig 80 for tracking inside pig pen 70.

Next, information processor 22 determines whether a predetermined requirement for the accumulated still pictures is satisfied (S25), and continues accumulation of still pictures until the predetermined requirement is satisfied (No in S25). The predetermined requirement indicates that a predetermined or larger number of still pictures enabling extraction of the top-side projection geometry and a predetermined or larger number of still pictures enabling extraction of the lateral projection geometry are accumulated, for example. In other words, the predetermined requirement is a requirement related with the number of still pictures.

When information processor 22 determines that the predetermined requirement for the accumulated still pictures is satisfied (Yes in S25), information processor 22 brings imaging device 10 into a zoom-in state having a zooming magnification higher than that during tracking imaging (S26). FIG. 8 is a diagram illustrating one example of the moving picture captured by imaging device 10 in the zoom-in state. As illustrated in FIG. 8, the moving picture captured by imaging device 10 in the zoom-in state includes an enlarged head portion of pig 80 for tracking. In other words, identification tag 90 is captured large. Thus, information processor 22 obtains the identification information of pig 80 for tracking from such a moving picture (S27). As illustrated in FIG. 8, when the moving picture includes color code 91 of identification tag 90, information processor 22 can obtain the identification information of pig 80 included in the moving picture. The zooming magnification may be optically changed depending on a change in arrangement of the lens included in imaging device 10, or may be changed by signal processing performed on the image.

Information processor 22 then stores the identification information obtained in step S27 in storage 24, in association with the still pictures accumulated in step S24 (S28). Hereinafter, the processing from steps S21 to S28 is repeated, and a set of the identification information, the still pictures enabling extraction of the top-side projection geometry, and those enabling extraction of the lateral projection geometry is stored for each of pigs 80 inside pig pen 70. By using the estimation method in FIG. 3 based on such a set of pieces of information, the weight of each of pigs 80 inside pig pen 70 can be daily stored in storage 24. Such information of the daily weight is transmitted to server 30, and is stored (managed) in server 30. The information of the daily weight is provided to information terminal 40, which can display a graph in FIG. 9 on the display screen. FIG. 9 is a diagram illustrating one example of the graph of the weight.

As described above, in Operational Example 1, animal information management system 100 performs the control for obtaining the identification information only when the requirement needed to estimate the weight of pig 80 is satisfied. For this reason, unnecessary execution of the processing for obtaining the identification information is suppressed. In other words, animal information management system 100 can efficiently identify individual pigs 80 using images.

### [Operational Example 2]

Imaging device 10 may perform tracking imaging of a plurality of pigs 80 (e.g., two pigs, and may be three or more pigs). FIG. 10 is a flowchart of Operational Example 2 in animal information management system 100 in such a case.

Initially, imaging device 10 captures the overall view of the inside of pig pen 70 as illustrated in FIG. 1 (S31). Information processor 22 detects any two or more pigs 80 by contour extraction performed on the moving picture during capturing (S32), and starts tracking imaging of the pigs detected by imaging device 10 (S33).

In tracking imaging, the pan angle, the tilt angle, and the zooming magnification of imaging device 10 are controlled such that the entire bodies of pigs 80 for tracking are included in the capturing range of imaging device 10, even when pigs 80 for tracking move inside pig pen 70. Such tracking imaging can be implemented by an existing algorithm such as an approach using artificial intelligence. The zooming magnification during tracking imaging is set such that pigs 80 are captured as large as possible within the capturing range (or in the still picture). The zooming magnification during tracking imaging is fixed, for example.

Next, information processor 22 accumulates the still pictures, which include pigs 80, in storage 24 during tracking imaging (S34). For example, information processor 22 performs contour extraction on pigs 80 for tracking in the moving picture obtained during tracking imaging, and when it is determined that at least one of contours extracted using a machine learning model or the like corresponds to the top-side projection geometry, information processor 22 stores the still pictures including such a contour in storage 24. Similarly, when it is determined that at least one of contours extracted using a machine learning model or the like corresponds to the lateral projection geometry, information processor 22 stores the still pictures including such a contour in storage 24. Each still picture is associated with temporary identification information indicating the projection geometry of the pig which can be extracted from the still picture, among the plurality of pigs 80.

Thus, in step S34, the still pictures enabling extraction of the top-side projection geometry and those enabling extraction of the lateral projection geometry are accumulated. Some weight estimation methods may need a projection geometry other than the top-side projection geometry and the lateral projection geometry in some cases. In such a case, another projection geometry may be accumulated in step S34.

In step S34, information processor 22 associates each of the still pictures with the capturing condition information of imaging device 10 when the still picture is captured, such as the pan angle, the tilt angle, and the zooming magnification. In step S34, information processor 22 may specify the coordinates information of pig 80 for tracking inside pig pen 70 based on the capturing condition information and the position information of pig 80 for tracking within the still picture, and may associate the still pictures with the specified coordinates information.

Next, information processor 22 determines that a predetermined requirement for the accumulated still pictures is satisfied (S35), and continues accumulation of still pictures until the predetermined requirement is satisfied (No in S35). The predetermined requirement indicates that a predetermined or larger number of still pictures enabling extraction of the top-side projection geometry and a predetermined or larger number of still pictures enabling extraction of the lateral projection geometry are accumulated for one of pigs 80 for tracking, for example. The determination in step S35 can be implemented by the temporary identification information described above.

When information processor 22 determines that the predetermined requirement for the accumulated still pictures is satisfied (Yes in S35), information processor 22 selects the one pig included in the predetermined or larger number of still pictures accumulated (S36), and brings imaging device 10 into the zoom-in state having a zooming magnification higher than that during tracking imaging such that the head of the selected pig is captured (S37). Information processor 22 then obtains the identification information of pig 80 selected from the moving picture captured by imaging device 10 in the zoom-in state (S38).

Information processor 22 then stores the identification information obtained in step S38 in storage 24, in association with the still pictures accumulated in step S34 (S39). At this time, among the still pictures accumulated in step S34, the still pictures not related with the selected pig 80 (for example, still pictures of pig 80 not selected, from which only the top-side projection geometry (or lateral projection geometry) can be extracted) are discarded. Hereinafter, the processing from steps S31 to step S39 is repeated, and a set of the identification information, the still pictures enabling extraction of the top-side projection geometry, and those enabling extraction of the lateral projection geometry is stored for each of pigs 80 inside pig pen 70.

As described above, in Operational Example 2, animal information management system 100 can accumulate the still pictures of pigs 80, which are candidates for estimation of the weight.

### [Modification 1]

Although imaging device 10 during tracking imaging has a fixed zooming magnification in the description of the embodiment above, the zooming magnification of imaging device 10 during tracking imaging may be varied. FIG. 11 is a diagram illustrating one example of a change in zooming magnification during tracking imaging.

As illustrated in FIG. 11, the zooming magnification of imaging device 10 during tracking imaging is basically a first zooming magnification. However, the zooming magnification of imaging device 10 in period T1 including capturing timing t of the still pictures is a second zooming magnification having a magnification higher than the first zooming magnification. In other words, in period T1 including capturing timing t of the still pictures during tracking imaging, information processor 22 causes the zooming magnification of imaging device 10 to be higher than that in another period T2 during tracking imaging. Such an operation can increase the size of pig 80 included in the still picture, thus ensuring a more accurate projection geometry.

### [Modification 2]

Although the predetermined requirement is related with the number of still pictures in the embodiment above, any other predetermined requirement can be specified. For example, the predetermined requirement may be a requirement related with the projection geometry of pig 80 included in a still picture.

For example, when the weight estimation method needs projection geometries of pig 80 captured from different n (where n is an integer of 2 or more) observing points and information processor 22 can distinguish and recognize m (where m is an integer larger than n) projection geometries, the predetermined requirement may be that still pictures from which n projection geometries among m projection geometries can be extracted are accumulated in storage 24.

### [Modification 3]

Although information processor 22 obtains the identification information of pig 80 using identification tag 90 in the embodiment above, such a method of obtaining the identification information is one example.

For example, each of pigs 80 may be ear-notched, and information processor 22 may obtain the identification information of pig 80 by detecting the ear notch included in the moving picture. FIG. 12 is a diagram illustrating one example of ear notching. The ear notch is one example of a characteristic portion of the pig.

As illustrated in FIG. 12, ear notching means that the edge of the ear is cut out. Pig 80 can be identified by the number of cutouts and the position(s) thereof. Information processor 22 can specify the position(s) and number of ear notches included in the moving picture by processing (e.g., contour extraction) to obtain the identification information of pig 80.

Alternatively, information processor 22 may obtain the identification information of pig 80 by detecting the silhouette of a characteristic portion of pig 80 included in the moving picture. The characteristic portion specifically indicates the outline of the nose, the eyes, or the head (face), the tail, the ear, or the horn (when the target animal has (a) horn(s)). In this case, for each of pigs 80 inside pig pen 70, the silhouettes of the characteristic portions are stored in storage 24 as referential silhouettes. The referential silhouettes are stored in association with the identification information.

Information processor 22 detects the silhouette of the characteristic portion of pig 80 included in the moving picture by image processing (e.g., contour extraction). Information processor 22 can compare the detected silhouette to the referential silhouettes stored in storage 24 to obtain the identification information associated with the most similar referential silhouette as the identification information of pig 80.

### [Effects]

In general, there is a room for examination of the method of managing the weight of pig 80 in association with the identification information of pig 80 by image processing. For example, the weights of pigs 80 can be estimated collectively by performing image processing on the still pictures including pigs 80. However, the sizes of the characteristic portions (or identification tags 90) of pigs 80 included in such still pictures are reduced, thus leading to difficulties in identifying animal individuals using the still pictures. In contrast, when a still picture is taken at a higher zooming magnification to recognize the characteristic portion of pig 80, the entire body of pig 80 cannot be captured, and thus the weight cannot be estimated.

This might lead to a configuration in which the zooming magnification is increased only when the identification information of pig 80 is obtained. However, if the zooming magnification is frequently changed in such a configuration, the identification information cannot be efficiently obtained.

Thus, animal information management system 100 includes storage 24 and information processor 22. Information processor 22 causes imaging device 10 to perform tracking imaging of pig 80, and accumulates still pictures including pig 80 in storage 24, the still pictures being obtained during tracking imaging. When the predetermined requirement for the accumulated still pictures is satisfied, information processor 22 brings imaging device 10 into a zoom-in state having a zooming magnification higher than that during tracking imaging, and obtains the identification information of pig 80 by causing imaging device 10 in the zoom-in state to capture an image of pig 80. Information processor 22 stores the obtained identification information in storage 24, in association with the accumulated still pictures. Pig 80 is one example of the animal.

In such animal information management system 100, unnecessary execution of the processing to obtain the identification information is suppressed by performing control for obtaining the identification information only when the requirement needed to estimate the weight of pig 80 is satisfied. In other words, animal information management system 100 can efficiently identify individual pigs 80 using images.

Moreover, for example, information processor 22 stores, in storage 24, items of position information each directly or indirectly indicating a position of pig 80 included in a corresponding one of the still pictures, each of the items of position information being stored in association with the corresponding one of the still pictures accumulated.

Such animal information management system 100 can store the identification information and the position information in association with the still pictures.

Moreover, for example, information processor 22 causes imaging device 10 to perform tracking imaging of a plurality of pigs 80, and accumulates still pictures including the plurality of pigs 80 in storage 24, the still pictures being obtained during tracking imaging. Information processor 22 obtains the identification information of one pig 80 among the plurality of pigs 80 by causing imaging device 10 in the zoom-in state to capture an image of one pig 80 when the predetermined requirement is satisfied, and stores the identification information of one pig 80 in storage 24, in association with the still pictures accumulated.

Such animal information management system 100 can accumulate the still pictures including the plurality of pigs 80.

Moreover, for example, in a period T1 including capturing timing t of the still pictures during tracking imaging, information processor 22 causes the zooming magnification of imaging device 10 to be higher than that in another period T2 during tracking imaging.

This operation increases the size of pig 80 included in the still pictures, thus obtaining still pictures in which pig 80 is more accurately captured.

Moreover, for example, identification tag 90 indicating the identification information of pig 80 by a sequence of mutually different adjacent colors is attached to pig 80. Information processor 22 obtains the identification information of pig 80, based on identification tag 90 captured by imaging device 10 in the zoom-in state. Identification tag 90 is one example of the identification member.

Such animal information management system 100 can obtain the identification information of pig 80 by detecting identification tag 90.

Moreover, for example, information processor 22 obtains the identification information of pig 80, based on a characteristic portion of pig 80 captured by imaging device 10 in the zoom-in state.

Such animal information management system 100 can obtain the identification information of pig 80 by detecting the characteristic portion of pig 80, such as an ear notch.

Moreover, for example, the predetermined requirement is a requirement related with the number of still pictures.

Such animal information management system 100 can efficiently identify individual pigs 80 using images, based on the requirement related with the number of still pictures.

Moreover, for example, the predetermined requirement is a requirement related with a projection geometry of pig 80 included in the still pictures.

Such animal information management system 100 can efficiently identify individual pigs 80 using images, based on the requirement related with the projection geometry of pig 80 included in the still pictures.

Moreover, for example, an animal information management method to be executed by a computer such as animal information management system 100 includes causing imaging device 10 to perform tracking imaging of pig 80; accumulating still pictures including pig 80 in storage 24, the still pictures being obtained during tracking imaging; bringing imaging device 10 into a zoom-in state having a zooming magnification higher than that during tracking imaging when a predetermined requirement for the accumulated still pictures is satisfied, and obtaining the identification information of pig 80 by causing imaging device 10 in the zoom-in state to capture an image of pig 80; and storing the identification information in storage 24, in association with the still pictures accumulated.

In such an animal information management method, unnecessary execution of the processing to obtain the identification information is suppressed by performing control for obtaining the identification information only when the requirement needed to estimate the weight of pig 80 is satisfied. In other words, the animal information management method can efficiently identify animal individuals using images.

### [Other embodiments]

The embodiment has been described as above, but this embodiment should not be construed as limitations to the present disclosure.

For example, although the animal information management system manages the information of the pig inside the pig pen, the animal information management system may manage information of a farm animal other than the pig, such as a cow. The animal information management system may be used in applications other than livestock raising, such as zoos.

In the embodiment above, the processing executed by a specific processor may be executed by another processor. The order of processings may be changed, or the processings may be executed concurrently.

In the embodiment above, the components may be implemented by executing software programs suitable for the components. Alternatively, the components may be implemented by a program executor such as a CPU or a processor, which reads out and executes the software programs recorded on a recording medium such as hard disk or a semiconductor memory.

Alternatively, the components may be implemented by hardware. For example, the component such as a controller may be a circuit (or an integrated circuit). These circuits may be formed into a single circuit as a whole, or may be formed as separate circuits. These circuits may be general-purpose circuits, or may be dedicated circuits.

The general or specific aspects of the present disclosure may be implemented by a system, a device, a method, an integrated circuit, a computer program, or a recording medium such as a computer-readable CD-ROM. The general or specific aspects of the present disclosure may be implemented by any combination of a system, a device, a method, an integrated circuit, a computer program, and a recording medium.

For example, the present disclosure may be implemented as an animal information management method executed by a computer such as an animal information management system, may be implemented as a program causing a computer to execute the animal information management method, or may be implemented as a non-transitory computer-readable recording medium having such a program recorded thereon.

Although the animal information management system is implemented by a plurality of devices in the embodiment above, the animal information management system may be implemented as a single device. When the animal information management system is implemented by a plurality of devices, the components included in the animal information management system described in the embodiment may be distributed to the plurality of devices in any manner.

Alternatively, the animal information management system may be implemented as a client server system. In this case, part or all of the processings performed by the information processing apparatus in the description of the embodiment is performed by the server.

Besides, the present disclosure also covers embodiments obtained by performing a variety of modifications conceived by persons skilled in the art on the embodiment above, or embodiments including any combination of the components and the functions included in the embodiment without departing the gist of the present disclosure.

### [Reference Signs List]

- 10: imaging device
- 20: information processing apparatus
- 22: information processor
- 24: storage
- 80: pig (animal)
- 90: identification tag (identification member)
- 100: animal information management system

## Claims

1. An animal information management system comprising:
a storage; and
an information processor,
wherein the information processor:
causes an imaging device to perform tracking imaging of an animal;
accumulates still pictures including the animal in the storage, the still pictures being obtained during the tracking imaging;
brings the imaging device into a zoom-in state having a zooming magnification higher than a zooming magnification during the tracking imaging when a predetermined requirement for the still pictures accumulated is satisfied, and obtains identification information of the animal by causing the imaging device in the zoom-in state to capture an image of the animal; and
stores the identification information in the storage, in association with the still pictures.

2. The animal information management system according to claim 1,
wherein the information processor stores, in the storage, items of position information each directly or indirectly indicating a position of the animal included in a corresponding one of the still pictures, each of the items of position information being stored in association with the corresponding one of the still pictures.

3. The animal information management system according to claim 1 or 2,
wherein the information processor:
causes the imaging device to perform tracking imaging of a plurality of animals;
accumulates still pictures including the plurality of animals in the storage, the still pictures being obtained during the tracking imaging;
obtains the identification information of one animal among the plurality of animals by causing the imaging device in the zoom-in state to capture an image of the one animal, when the predetermined requirement is satisfied; and
stores the identification information of the one animal in the storage, in association with the still pictures accumulated.

4. The animal information management system according to any one of claims 1 to 3,
wherein in a period including a capturing timing of the still pictures during the tracking imaging, the information processor causes the zooming magnification of the imaging device to be higher than a zooming magnification in another period during the tracking imaging.

5. The animal information management system according to any one of claims 1 to 4,
wherein an identification member indicating the identification information of the animal by a sequence of mutually different adjacent colors is attached to the animal, and
the information processor obtains the identification information of the animal, based on the identification member captured by the imaging device in the zoom-in state.

6. The animal information management system according to any one of claims 1 to 4,
wherein the information processor obtains the identification information of the animal, based on a characteristic portion of the animal captured by the imaging device in the zoom-in state.

7. The animal information management system according to any one of claims 1 to 6,
wherein the predetermined requirement is a requirement related with a total number of the still pictures.

8. The animal information management system according to any one of claims 1 to 6,
wherein the predetermined requirement is a requirement related with a projection geometry of the animal included in the still pictures.

9. An animal information management method to be executed by a computer,
the animal information management method comprising:
causing an imaging device to perform tracking imaging of an animal;
accumulating still pictures including the animal in a storage, the still pictures being obtained during the tracking imaging;
bringing the imaging device into a zoom-in state having a zooming magnification higher than a zooming magnification during the tracking imaging when a predetermined requirement for the still pictures is satisfied, and obtaining identification information of the animal by causing the imaging device in the zoom-in state to capture an image of the animal; and
storing the identification information in the storage, in association with the still pictures accumulated.

10. A program causing a computer to execute the animal information management method according to claim 9.
